(19) Europäisches Patentamt / European Patent Office / Office européen des brevets

(11) **EP 3 585 240 B1**

(12) **EUROPÄISCHE PATENTSCHRIFT**

(45) Veröffentlichungstag und Bekanntmachung des Hinweises auf die Patenterteilung:
**05.07.2023   Patentblatt 2023/27**

(21) Anmeldenummer: **18708052.8**

(22) Anmeldetag: **06.02.2018**

(51) Internationale Patentklassifikation (IPC):
**A61B 1/00** *(2006.01)*    **A61B 1/12** *(2006.01)*
**B08B 9/032** *(2006.01)*

(52) Gemeinsame Patentklassifikation (CPC):
**A61B 1/00057; A61B 1/125; B08B 9/0325;**
B08B 9/0328

(86) Internationale Anmeldenummer:
**PCT/EP2018/052880**

(87) Internationale Veröffentlichungsnummer:
**WO 2018/153653 (30.08.2018 Gazette 2018/35)**

(54) **AUFBEREITUNGSVORRICHTUNG UND VERFAHREN ZUM BETREIBEN EINER AUFBEREITUNGSVORRICHTUNG ZUM REINIGEN UND/ODER DESINFIZIEREN EINES MEDIZINISCHEN INSTRUMENTS**

PREPARATION DEVICE AND METHOD FOR OPERATING A PREPARATION DEVICE FOR CLEANING AND/OR DISINFECTING A MEDICAL INSTRUMENT

DISPOSITIF DE PRÉPARATION ET PROCÉDÉ DE FONCTIONNEMENT D'UN DISPOSITIF DE PRÉPARATION DESTINÉ AU NETTOYAGE ET/OU À LA DÉSINFECTION D'UN INSTRUMENT MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.02.2017   DE 102017202869**

(43) Veröffentlichungstag der Anmeldung:
**01.01.2020   Patentblatt 2020/01**

(73) Patentinhaber: **Olympus Winter & Ibe GmbH**
**22045 Hamburg (DE)**

(72) Erfinder:
• **ERDMANN, Niklas**
**21147 Hamburg (DE)**

• **ESCHBORN, Sascha**
**22926 Ahrensburg (DE)**
• **WEIS, Antonia**
**22047 Hamburg (DE)**

(74) Vertreter: **Seemann & Partner Patentanwälte mbB**
**Raboisen 6**
**20095 Hamburg (DE)**

(56) Entgegenhaltungen:
**US-A1- 2010 078 047    US-A1- 2011 097 248**
**US-A1- 2012 019 807**

**Beschreibung**

Die Erfindung betrifft ein Verfahren zum Betreiben einer Aufbereitungsvorrichtung zum Reinigen und/oder Desinfizieren eines medizinischen Instruments, das wenigstens einen Kanal aufweist, wobei eine Fluidleitung vorgesehen ist, die mit dem wenigstens einen Kanal verbunden ist oder wird.

Die Erfindung betrifft ferner eine Aufbereitungsvorrichtung zur Reinigung und/oder Desinfizierung von medizinischen Instrumenten mit einem Fluidbehälter für ein Aufbereitungsfluid und einem Aufbereitungsgerät, wobei das Aufbereitungsgerät einen Aufbereitungsraum aufweist, in dem ein medizinisches Instrument zur Aufbereitung einbringbar ist oder eingebracht ist, wobei wenigstens ein Kanal des medizinischen Instruments mit einer Fluidleitung verbindbar ist, wobei die Fluidleitung dazu dient, das Aufbereitungsfluid zum wenigstens einen Kanal zu transportieren.

Die Aufbereitung von medizinischen Instrumenten, die Kanäle umfassen, beispielsweise Endoskope, sowohl flexible als auch starre Endoskope, gliedert sich in die Aufbereitung der Außenflächen und die Aufbereitung der Innenkanäle bzw. Kanäle. Die Aufbereitung der Kanäle kann im Hinblick auf das hygienische Ergebnis problematisch sein, da diese nicht optisch kontrolliert werden können. Hierzu werden Schläuche oder Fluidleitungen beispielsweise teilweise mit Adaptern an die Anschlüsse des medizinischen Instruments bzw. des Endoskops angeschlossen und unter Druck wird ein Aufbereitungsfluid über diese Schläuche durch die Innenkanäle bzw. Kanäle des Endoskops geleitet, um diese zu reinigen und zu desinfizieren. Falls die Adapter nicht oder falsch angeschlossen wurden, können Probleme auftreten. Zudem kann ein oder können mehrere Kanäle des Endoskops bzw. des medizinischen Instruments blockiert sein, so dass kein oder zu wenig Fluid durch den jeweiligen Kanal fließen kann und damit das Aufbereitungsergebnis nicht sichergestellt werden kann.

Daher ist es im Stand der Technik bekannt, einen Volumenstrom, der durch die einzelnen Kanäle eines medizinischen Instruments fließt, zu messen und mit einem Referenzvolumenstrom abzugleichen. Dies kann bei einer Aufbereitungsvorrichtung der Anmelderin des Namens ETD3/4 über eine Messung der Geschwindigkeit von Ionen im Fluid geschehen oder aber über eine Messung des Kanaldrucks, beispielsweise in der ETD Double der Anmelderin. Über den Kanaldruck kann der Volumenstrom auch berechnet werden, was zur Überprüfung der Adaptierung und der Durchgängigkeit des jeweiligen Kanals führt.

Bei der Bestimmung der Geschwindigkeit von Ionen im Prozessfluid können Probleme auftreten, wenn ein Prozessschritt durchgeführt werden soll, in dem keine chemischen Bestandteile zu sauberem beispielsweise voll entsalztem Wasser hinzugefügt werden dürfen. Die Bestimmung der Fließgeschwindigkeit über das Vorsehen eines entsprechenden Drucks im Kanal ist relativ zeitaufwendig.

US 2011/0097248 A1 zeigt eine automatisierte Endoskopreinigungs- und Desinfektionsvorrichtung. Mittels der Vorrichtung wird ein Fluid in Endoskopöffnungen eingeleitet und ein Zustand des Fluids gemessen, um eine Abschätzung der Reinigungsleistung durch das Fluid zu gewinnen.

In US 2010/0078047 A1 ist ein Verfahren, eine Zusammensetzung und eine Vorrichtung zum Reinigen von dünnen Kanälen, beispielsweise in Endoskopen, offenbart. Dazu wird eine Flüssigkeit und ein Gas unter verschiedenen Flusszuständen durch den Kanal gespült. Es ist Aufgabe der vorliegenden Erfindung, ein kostengünstiges, effizientes und wenig Zeit benötigendes Verfahren zum Betreiben einer Aufbereitungsvorrichtung zum Reinigen und/oder Desinfizieren eines medizinischen Instruments anzugeben und eine entsprechende kostengünstige und effiziente Aufbereitungsvorrichtung sowie ein entsprechendes Modul zur Verwendung in einer Aufbereitungsvorrichtung zur Reinigung und/oder Desinfizierung von medizinischen Instrumenten.

Gelöst wird diese Aufgabe durch ein Verfahren zum Betreiben einer Aufbereitungsvorrichtung zum Reinigen und/oder Desinfizieren eines medizinischen Instruments gemäß unabhängigem Anspruch 1, das wenigstens einen Kanal aufweist, wobei eine Fluidleitung vorgesehen ist, die mit dem wenigstens einen Kanal verbunden ist oder wird, das dadurch weitergebildet ist, dass zur Bestimmung des Volumenstroms einer in der Fluidleitung strömenden Flüssigkeit Gasblasen in die Flüssigkeit eingelassen sind oder werden, wobei die Geschwindigkeit der Gasblasen bestimmt wird und anhand der Geschwindigkeit der Gasblasen auf den Volumenstrom geschlossen wird, wobei die Geschwindigkeit der Gasblasen über eine Bildverarbeitung von wenigstens zwei zeitlich aufeinander folgenden aufgenommenen Bildern eines Ausschnitts der Fluidleitung bestimmt wird, wobei zur Kontrasterhöhung Licht auf die Gasblasen geleuchtet wird, wobei die Leuchtrichtung annähernd parallel oder antiparallel zur Fließrichtung der Flüssigkeit ist.

Hierdurch ist eine effiziente Durchflusskontrolle möglich und zudem die Überprüfung auf Verstopfung in einem Kanal des medizinischen Instruments. Durch die Verwendung von Gasblasen, die in die Flüssigkeit eingebracht werden, wird verhindert, dass durch andere Materialien, die gemäß dem Stand der Technik in die Flüssigkeit eingelassen werden, Rückstände in dem medizinischen Instrument verbleiben können. Die Gasblasen werden somit als Art Marker verwendet, um insbesondere ein sogenanntes Particle Image Velocimetry Verfahren anzuwenden. Hierbei werden insbesondere, vorzugsweise mit einer Kamera, die in einem Fluid eingebrachten Markerpartikel erfasst und in konsekutiven Bildern ausgewertet. Zum Einbringen von Gasblasen, beispielsweise Luftblasen, kann eine Druckluftzufuhr, eine Druckflasche oder eine Pumpe verwendet werden. Es kann auch ein anderes Gas, beispielsweise ein Edelgas, aus einer Druckflasche unter Zwischenschaltung eines Dosierventils in die Fluidleitung eingebracht werden.

Erfindungsgemäß wird die Geschwindigkeit der Gasblasen über eine Verarbeitung von wenigstens zwei zeitlich aufeinander folgenden aufgenommenen Bildern eines Ausschnitts der Fluidleitung bestimmt.

Ferner wird zur Kontrasterhöhung Licht, insbesondere infrarotes Licht, auf die Gasblasen geleuchtet. Hierdurch werden die als Marker verwendeten Gasblasen in den aufgenommenen Bildern betont bzw. der Kontrast dieser Marker erhöht. Hierzu wird eine Leuchtrichtung vorgesehen, die annähernd parallel oder antiparallel zur Fließrichtung der Flüssigkeit ist. Vorzugsweise ist ein Einfallswinkel der Lichtstrahlen vorgesehen, der zwischen 1° und 30°, insbesondere zwischen 2° und 15° und besonders vorzugsweise zwischen 5° und 10° zur Fließrichtung der Flüssigkeit vorgesehen bzw. zu einer Mittelachse der Fluidleitung ist. Durch Verwenden von vorzugsweise Infrarotlicht erscheinen die Gasblasen in aufgenommenen Bildern nahezu weiß, da Gasblasen das Licht in alle Richtung streuen, während andere Objekte im Bild das Licht nur brechen und dadurch nicht in Richtung einer Kamera reflektieren.

Die Kamera, die im Rahmen der Erfindung auch als Bildaufnahmevorrichtung bezeichnet werden kann, ist für verschiedene Frequenzen empfindlich. Insbesondere ist eine Infrarotempfindlichkeit und/oder eine Empfindlichkeit bzw. Aufnahmemöglichkeit bei sichtbarem Licht gegeben.

Vorzugsweise wird die Geschwindigkeit der Gasblasen in der Nähe einer Wand der Fluidleitung bestimmt, wobei hierzu insbesondere die Geschwindigkeit der Gasblasen in einem Abstand von der Mitte der Fluidleitung zu einer Wand bestimmt wird, wobei der Abstand $\geq$ (größer oder gleich) $R/2^{1/2}$ ist, wobei R der innere Radius der Fluidleitung oder ein Abstand einer Mittelachse zu einer Wand der Fluidleitung ist. $R/2^{1/2}$ entspricht

$$\frac{R}{\sqrt{2}}.$$

Vorzugsweise wird zum Bestimmen der Geschwindigkeit eine Fouriertransformation der zeitlich aufeinander folgenden aufgenommenen Bilder durchgeführt, wobei insbesondere über eine Phasenkorrelation ein Bewegungsvektor der Gasblasen ermittelt wird. Hierbei kann die Geschwindigkeit der Gasblasen bzw. Markerpartikel im Fluid über einen Phasenkorrelationsalgorithmus im Fourierraum aus zwei oder mehreren aufeinanderfolgenden Bildern abgeleitet werden. Über die Geschwindigkeit der Gasblasen kann dann rechnerisch auf den Volumenstrom rückgeschlossen werden. Hierbei sollte zwischen einem laminaren und einem turbulenten Fluss unterschieden werden. Zudem ist die Position der Gasblasen im Schlauch relativ zur Schlauchmitte bzw. zur Wand vorzugeben oder zu messen. Ein entsprechendes Verfahren kann an jedem Fluidleitungsanschluss eines Aufbereitungsgerätes oder bei jeder Fluidleitung durchgeführt werden, und zwar mit jeweils einer Kamera, Es kann allerdings auch eine Kamera für mehrere Messstellen verwendet werden, wobei dann die Kamera mehrere Messbereiche bzw. Abschnitte von verschiedenen Fluidleitungen erfassen kann oder aber die Kamera entsprechend zu den jeweiligen Fluidleitungen gerichtet werden kann.

Gemäß der Erfindung wurde zur Bestimmung von Volumenströmen in einem Bereich von 500 ml/min bis 2.900 ml/min ein Messfehler von 10% und weniger erzielt. Das erfindungsgemäße Verfahren funktioniert auch bei geringeren Volumenströmen von beispielsweise 350 ml/min.

Die Aufgabe wird ferner gelöst durch eine Aufbereitungsvorrichtung zur Reinigung und/oder Desinfizierung von medizinischen Instrumenten gemäß unabhängigem Anspruch 4, mit einem Fluidbehälter für ein Aufbereitungsfluid und einem Aufbereitungsgerät, wobei die Aufbereitungsvorrichtung eine Fluidleitung aufweist, wobei das Aufbereitungsgerät einen Aufbereitungsraum aufweist, in dem ein medizinisches Instrument zur Aufbereitung einbringbar ist oder eingebracht ist, wobei wenigstens ein Kanal des medizinischen Instruments mit der Fluidleitung verbindbar ist, wobei die Fluidleitung dazu dient, das Aufbereitungsfluid zu wenigstens einem Kanal zu transportieren, die dadurch weitergebildet ist, dass eine Blaseneinleitvorrichtung vorgesehen ist, mittels der Gasblasen in die Fluidleitung einbringbar sind, wobei zudem eine Gasblasengeschwindigkeitsbestimmungsvorrichtung vorgesehen ist. Hierdurch kann eine sehr kostengünstige und sichere Aufbereitungsvorrichtung zur Reinigung und/oder Desinfizierung von medizinischen Instrumenten zur Verfügung gestellt werden, wobei die Gasblasengeschwindigkeitsbestimmungsvorrichtung eine Beleuchtungsvorrichtung aufweist, wobei die Beleuchtungsvorrichtung Licht annähernd parallel oder antiparallel zu einer Mittelachse der Fluidleitung in die Fluidleitung einstrahlt.

Vorzugsweise weist die Aufbereitungsvorrichtung eine Adaptervorrichtung auf, an die eine Fluidleitung anschließbar ist und die dazu dient, das Fluid zu dem wenigstens einen Kanal zu leiten. Bei mehreren Kanälen im medizinischen Instrument kann die Adaptervorrichtung auch dazu dienen, Fluid auf die Kanäle zu verteilen.

Vorzugsweise weist die Gasblasengeschwindigkeitsbestimmungsvorrichtung eine Kamera zur Aufnahme von zeitlich aufeinander folgenden Bildern wenigstens eines Abschnitts der Fluidleitung auf.

Vorzugsweise ist die Fluidleitung in dem Abschnitt transparent, wodurch es möglich ist, dass die Kamera außerhalb der Fluidleitung angeordnet ist. Insbesondere ist der Abschnitt insofern transparent, als dieses für sichtbares Licht oder für Infrarotlicht oder für ultraviolette Strahlung transparent ist. Unter transparent wird auch eine leichte Milchigkeit verstanden, wobei wesentlich ist, dass die Gasblasen noch gut erkennbar sind. Die Kamera ist vorzugsweise ausgebildet, sichtbares Licht, Infrarotlicht oder ultraviolettes Licht zu detektieren.

Die Gasblasengeschwindigkeitsbestimmungsvorrichtung weist eine Beleuchtungsvorrichtung auf, wobei die Beleuchtungsvorrichtung insbesondere Licht im infraroten Bereich emittiert.

Die Beleuchtungsvorrichtung strahlt Licht annähernd parallel oder antiparallel zu einer Mittelachse der Fluidleitung in die Fluidleitung ein. Hierdurch kann der Kontrast der Gasblasen in dem Fluid für die aufzunehmenden Bilder durch die Kamera deutlich erhöht werden. Vorzugsweise umfasst der Abschnitt wenigstens einen Bereich, der von einer Mittelachse der Fluidleitung $\geq R/2^{1/2}$ entfernt ist, wobei R ein Radius der Fluidleitung oder ein Abstand der Mittelachse zu einer Wand der Fluidleitung ist.

Wenn die Gasblasengeschwindigkeitsbestimmungsvorrichtung ein Computersystem umfasst, auf dem insbesondere eine Fouriertransformation von zeitlich aufeinanderfolgenden aufgenommenen Bildern durchführbar ist, wobei insbesondere über eine Phasenkorrelation ein Bewegungsvektor der Gasblasen ermittelbar ist, kann auf besonders sichere Weise der Volumenstrom der Flüssigkeit in der Fluidleitung bestimmt werden.

Erfindungsgemäß werden Gasblasen zur Bestimmung des Volumenstroms eines Aufbereitungsfluids in einem Kanal eines aufzubereitenden medizinischen Instruments verwendet. Hierzu kann das erfindungsgemäße Verfahren, die erfindungsgemäße Aufbereitungsvorrichtung und das im Folgenden noch beschriebene erfindungsgemäße Volumenstrombestimmungsmodul verwendet werden.

Der Volumenstrom wird anhand der Geschwindigkeit der Gasblasen bestimmt.

Die Aufgabe wird ferner durch ein Volumenstrombestimmungsmodul zur Verwendung in oder mit einer Aufbereitungsvorrichtung zum Reinigen und/oder Desinfizieren eines medizinischen Instruments gemäß unabhängigem Anspruch 11 gelöst, wobei das Volumenstrombestimmungsmodul eine Fluidleitung oder einen Abschnitt einer Fluidleitung, eine Blaseneinleitvorrichtung zum Einleiten von Gasblasen in die Fluidleitung oder den Abschnitt der Fluidleitung, und eine Gasblasengeschwindigkeitsbestimmungsvorrichtung umfasst, wobei die Gasblasengeschwindigkeitsbestimmungsvorrichtung eine Beleuchtungsvorrichtung aufweist, wobei die Beleuchtungsvorrichtung Licht annähernd parallel oder antiparallel zu einer Mittelachse der Fluidleitung in die Fluidleitung einstrahlt.

Das erfindungsgemäße Volumenstrombestimmungsmodul wird vorzugsweise in einer Aufbereitungsvorrichtung zum Reinigen und/oder Desinfizieren eines medizinischen Instruments verwendet. Mittels des erfindungsgemäßen Volumenstrombestimmungsmoduls kann das erfindungsgemäße Verfahren oder ein bevorzugtes Verfahren angewendet werden.

Vorzugsweise weist die Gasblasengeschwindigkeitsbestimmungsvorrichtung eine Kamera zur Aufnahme von zeitlich aufeinanderfolgenden Bildern wenigstens eines Abschnitts der Fluidleitung auf. Ferner vorzugsweise ist die Fluidleitung oder der Abschnitt der Fluidleitung mit einem Abschnitt oder einem Teil versehen, der transparent ist. Die Gasblasengeschwindigkeitsbestimmungsvorrichtung weist eine Beleuchtungsvorrichtung auf, wobei die Beleuchtungsvorrichtung insbesondere Licht im infraroten Bereich emittiert. Das Licht wird im Wesentlichen parallel oder antiparallel zu einer Mittelachse der Fluidleitung in die Fluidleitung eingestrahlt werden, und zwar insbesondere in einem vorstehend schon benannten Winkel zur Mittelachse der Fluidleitung oder zur Fluidleitung. Zudem können auch die weiteren bevorzugten Maßnahmen, die auch im Hinblick auf die Erfindung zur Aufbereitungsvorrichtung schon genannt sind, bei dem Volumenstrombestimmungsmodul vorgesehen sein.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1 eine schematische Darstellung einer erfindungsgemäßen Aufbereitungsvorrichtung zur Reinigung und/oder Desinfizierung von medizinischen Instrumenten,

Fig. 2 eine schematische Schnittdarstellung eines Abschnitts einer Fluidleitung, in der mögliche Strömungsprofile dargestellt sind.

In den Zeichnungen sind jeweils gleiche oder gleichartige Elemente und/oder Teile mit denselben Bezugsziffern versehen, so dass von einer erneuten Vorstellung jeweils abgesehen wird.

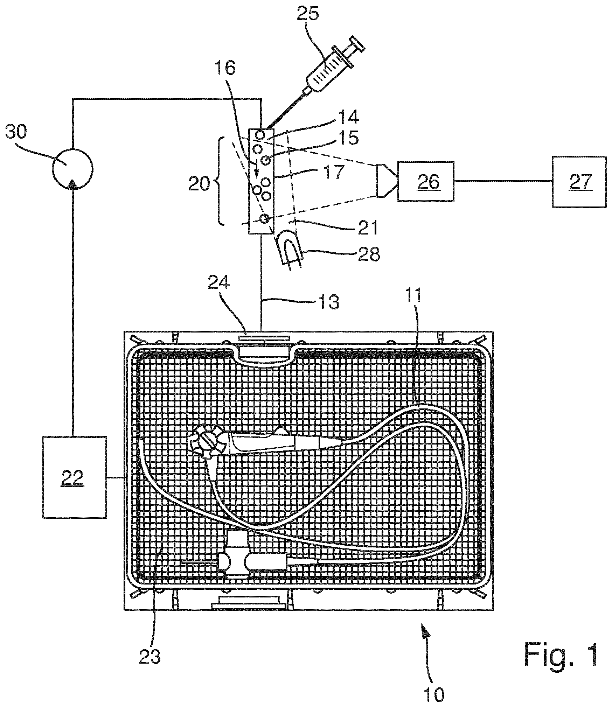

Figur 1 zeigt schematisch eine erfindungsgemäße Aufbereitungsvorrichtung zur Reinigung und/oder Desinfizierung von medizinischen Instrumenten 11. Es ist ein Aufbereitungsgerät 10 vorgesehen, das in einem Aufbereitungsraum 23 einen Korb zur Aufnahme eines Endoskops 11 aufweist. Das Endoskop 11 umfasst einen oder mehrere dort in Figur 1 nicht dargestellte Kanal 12 oder Kanäle 12, der oder die zu reinigen oder zu desinfizieren ist oder sind. Die Kanäle 12 werden mit einer Flüssigkeit gespült, die über nicht dargestellte Leitungen von einer Adaptervorrichtung 24 zur Verfügung gestellt werden. Eine entsprechende Adaptervorrichtung bzw. Verteilvorrichtung ist in der WO 2011/149 539 A1 dargestellt.

Die Flüssigkeit gelangt über Fluidleitungen 13 vorzugsweise in einem Kreislauf von einem Fluidbehälter 22 angetrieben durch eine Pumpe 30 zu der Adaptervorrichtung 24 in den entweder teilweise direkt in den Aufbereitungsraum 23 und/oder über die nicht dargestellten Leitungen in die Kanäle 12 des Endoskops 11 und von den Enden der Kanäle 12 in den Aufbereitungsraum 23 und von dem Aufbereitungsraum 23 wieder in den Fluidbehälter 22. Es kann auch eine Reinigung der Flüssigkeit, die in dem Kreislauf geführt wird, vorgesehen sein oder alternativ kein Kreislauf, sondern die ständige Zufuhr von frischer sauberer Flüssigkeit.

Erfindungsgemäß ist nun vorgesehen, über eine schematisch als Spritze dargestellte Blaseneinleitvorrichtung 25, Gasblasen 15 in die Fluidleitung 13 einzubringen, und zwar in die Flüssigkeit 14. Die Flüssigkeit 14 bewegt sich in Fließrichtung 16 in der Fluidleitung 13 fort. Die Gasblasen 15 werden in einem Abschnitt 20 durch eine Kamera 26 aufgenommen und es wird die Geschwindigkeit der Gasblasen 15 in dem Abschnitt 20 durch Auswertung von aufeinanderfolgenden Bildern der Kamera in einem Computersystem 27 bestimmt. Dies wird im Folgenden noch näher erläutert. Ein Computersystem 27 kann im Rahmen der Erfindung jede Vorrichtung sein, die digitale Berechnungen durchführt.

Zur Erhöhung des Kontrastes der Gasblasen 15 in dem Fluid 14 ist eine Beleuchtungsvorrichtung 28 vorgesehen, die im Wesentlichen parallel oder antiparallel zur Fließrichtung 16, insbesondere infrarotes, Licht in den Abschnitt 20 bzw. Ausschnitt 20 der Fluidleitung strahlt. Das Licht ist mit der Bezugsziffer 21 versehen. Die gestrichelten Linien um die Bezugsziffer 21 deuten den Divergenzwinkel der Strahlen der Beleuchtungsvorrichtung 28 an und entsprechende gestrichelte Linien von der Kamera 26 deuten den Bildausschnitt der Kamera 26 an.

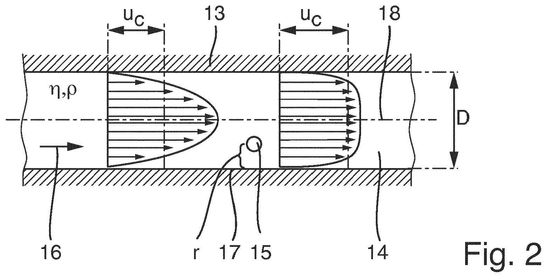

Im Zusammenhang mit Figur 2 soll das Messprinzip näher erläutert werden. Das Messprinzip zur Ermittlung der Geschwindigkeit der Gasblasen 15 bzw. das Volumenstromermittlungsverfahren basiert auf einem Particle Image Velocimetry Verfahren

Figur 2 zeigt hierzu eine schematische Schnittdarstellung einer Fluidleitung 13, in der Geschwindigkeitsprofile von Fluiden dargestellt sind. Die Fluidleitung 13 hat eine Wand 17 und einen Durchmesser D. In der Fluidleitung 13 ist eine Flüssigkeit 14 vorgesehen, die in Fließrichtung 16 fließt und eine Dichte $\rho$ sowie eine Viskosität $\eta$ hat. Zudem ist in Figur 2 ein Geschwindigkeitsprofil bei laminarer Strömung gezeigt, und zwar links in Figur 2, und ein Geschwindigkeitsprofil bei turbulenter Strömung, und zwar rechts in Figur 2. Die mittlere Geschwindigkeit $u_c$ ist auch dargestellt.

Bei der Particle Image Velocimetry werden bewegliche Objekte in einer Bilderserie aufgenommen. Aufgrund der Bewegung der Objekte oder eines Objektes in konsekutiven Bildern einer Serie von Bildem werden ein Bewegungsvektor sowie die Bewegungsgeschwindigkeit des Objekts ermittelt. Hierzu sind sichtbare Objekte in einem Fluidfluss vorhanden. Erfindungsgemäß werden hierzu Gasblasen oder Luftblasen verwendet.

Der Bewegungsvektor dieser Gasblasen wird bestimmt, um eine Flussgeschwindigkeit zu ermitteln. Hierzu wird als Beispiel eine Phasenkorrelation verwendet, die als Basis eine Kreuzkorrelation aufweist. Dieses basiert darauf, dass verschobene Signale im Fourierraum die gleiche Amplitude, jedoch unterschiedliche Phasen aufweisen. Bei der Particle Image Velocimetry wird die konkrete Geschwindigkeit an einer Stelle in der Strömung bzw. in dem Fluidfluss gemessen und hieraus kann eine mittlere Strömungsgeschwindigkeit berechnet werden. Die mittlere Strömungsgeschwindigkeit, mit der der Volumenstrom berechnet werden kann, hängt davon ab, ob eine laminare oder eine turbulente Strömung vorliegt. Hierzu ist Figur 2 zu berücksichtigen. Eine Basisversion der Fig. 2 ist aus dem Lehrbuch "Strömungsmechanik - Einführung in die Physik von technischen Strömungen", Vieweg + Teubner 2008, entnommen.

Bei einer laminaren Strömung ist der Fluss schichtartig, da die Bewegungsgeschwindigkeit der Fluidteilchen nur in Richtung der Rohrachse bzw. der Fluidleitungsachse liegt. Die Fluidteilchen bzw. Gasblasen haben daher eine feste Position relativ zur Mittelachse 18 der Fluidleitung 13. Das Maximum der Geschwindigkeit liegt mittig im Rohr auf der Achse 18 und die niedrigste Geschwindigkeit an der Wand 17 (siehe linke Seite der Fig 2).

Bei turbulenten Strömungen gibt es eine konstante Verwirbelung des Fluids im Strömungsquerschnitt, so dass über den gesamten Querschnitt im Wesentlichen die gleiche Strömungsgeschwindigkeit herrscht. Diese entspricht in einer Näherung der mittleren Strömungsgeschwindigkeit (siehe rechte Seite der Fig. 2). Um zwischen einer laminaren und einer turbulenten Strömung zu unterscheiden, wird die Reynoldszahl angeführt. Sofern die Reynoldszahl über der Grenze von 2.300 liegt, handelt es sich bei der Strömung um einen turbulenten Vorgang. Bei geringerer Reynoldszahl ist die Strömung laminar. Bei niedrigen Strömungsgeschwindigkeiten ist somit mit einer laminaren Strömung zu rechnen und bei hohen Strömungsgeschwindigkeiten mit einer turbulenten Strömung. Über die Querschnittsfläche der Fluidleitung 13 und die bestimmte mittlere Geschwindigkeit $u_c$ kann der Volumenstrom bestimmt werden. Ein typischer Grenzvolumenstrom, bei dem der Übergang von einer laminaren zu einer turbulenten Strömung stattfindet, ist bei Fluidleitungen, die zur Reinigung und/oder Desinfizierung von medizinischen Instrumenten wie Endoskopen dienen, bei ungefähr 870 ml/min. Bei den üblichen verwendeten medizinischen Instrumenten liegt der Grenzvolumenstrom bei 400 ml/min bis 1000 ml/min, insbesondere 600 ml/min bis 900 ml/min.

Vorzugsweise wird die Flussmessung bzw. die Messung der Geschwindigkeit der Gasblasen in der Nähe der Wand 17 durchgeführt. Der Abstand der Messung sollte hierbei vorzugsweise größer oder gleich $R/2^{1/2}$ sein, wobei R der Radius der Fluidleitung 13 ist bzw. die Hälfte des Durchmessers D ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

Bezugszeichenliste

| | |
|---|---|
| 10 | Aufbereitungsgerät |
| 11 | Endoskop |
| 12 | Kanal |
| 13 | Fluidleitung |
| 14 | Flüssigkeit |
| 15 | Gasblase |
| 16 | Fließrichtung |
| 17 | Wand |
| 18 | Mittelachse |
| 20 | Ausschnitt |
| 21 | Licht |
| 22 | Fluidbehälter |
| 23 | Aufbereitungsraum |
| 24 | Adaptervorrichtung |
| 25 | Blaseneinleitvorrichtung |
| 26 | Kamera |
| 27 | Computersystem |
| 28 | Beleuchtungsvorrichtung |
| 30 | Pumpe |

| | |
|---|---|
| u | Geschwindigkeit |
| $u_c$ | mittlere Geschwindigkeit |
| D | Durchmesser |
| R | Radius |
| r | Abstand zur Wand |
| η | Viskosität |
| ρ | Dichte |

**Patentansprüche**

1. Verfahren zum Betreiben einer Aufbereitungsvorrichtung (10) zum Reinigen und/oder Desinfizieren eines medizinischen Instruments (11), das wenigstens einen Kanal (12) aufweist, wobei eine Fluidleitung (13) vorgesehen ist, die mit dem wenigstens einen Kanal (12) verbunden ist oder wird, wobei zur Bestimmung des Volumenstroms einer in der Fluidleitung (13) strömenden Flüssigkeit (14) Gasblasen (15) in die Flüssigkeit (14) eingelassen sind oder werden, wobei die Geschwindigkeit (u) der Gasblasen (15) bestimmt wird und anhand der Geschwindigkeit (u) der Gasblasen (15) auf den Volumenstrom geschlossen wird, **dadurch gekennzeichnet, dass** die Geschwindigkeit (u) der Gasblasen (15) über eine Bildverarbeitung von wenigstens zwei zeitlich aufeinander folgenden aufgenommenen Bildern eines Ausschnitts (20) der Fluidleitung (13) bestimmt wird, wobei zur Kontrasterhöhung Licht (21) auf die Gasblasen (15) geleuchtet wird, wobei die Leuchtrichtung annähernd parallel oder antiparallel zur Fließrichtung (16) der Flüssigkeit (14) ist.

2. Verfahren nach Anspruch 1, **dadurch gekennzeichnet, dass** die Geschwindigkeit (u) der Gasblasen (15) in der Nähe einer Wand (17) der Fluidleitung (13) bestimmt wird, wobei hierzu insbesondere die Geschwindigkeit (u) der Gasblasen (15) in einem Abstand von der Mitte (18) der Fluidleitung (13) zu einer Wand (17) bestimmt wird, wobei der Abstand $\geq R/2^{1/2}$ ist, wobei R der innere Radius der Fluidleitung (13) oder ein Abstand einer Mittelachse (18) zu einer Wand (17) der Fluidleitung (13) ist.

3. Verfahren nach Anspruch 1 oder 2, **dadurch gekennzeichnet, dass** zum Bestimmen der Geschwindigkeit (u) eine Fouriertransformation der zeitlich aufeinander folgenden aufgenommenen Bilder durchgeführt wird, wobei insbesondere über eine Phasenkorrelation ein Bewegungsvektor der Gasblasen (15) ermittelt wird.

4. Aufbereitungsvorrichtung zur Reinigung und/oder Desinfizierung von medizinischen Instrumenten (11) mit einem Fluidbehälter (22) für ein Aufbereitungsfluid (14) und einem Aufbereitungsgerät (10), wobei die Aufbereitungsvorrichtung eine Fluidleitung (13) aufweist, wobei das Aufbereitungsgerät (10) einen Aufbereitungsraum (23) aufweist, in dem ein medizinisches Instrument (11) zur Aufbereitung einbringbar ist oder eingebracht ist, wobei wenigstens ein Kanal (12) des medizinischen Instruments (11) mit der Fluidleitung (13) verbindbar oder verbunden ist, wobei die Fluidleitung (13) dazu dient, das Aufbereitungsfluid (14) zum wenigstens einen Kanal (12) zu transportieren, wobei eine Blaseneinleitvorrichtung (25) vorgesehen ist, mittels der Gasblasen (15) in die Fluidleitung (13) einbringbar sind, wobei zudem eine Gasblasengeschwindigkeitsbestimmungsvorrichtung (26, 27, 28) vorgesehen ist, **dadurch gekennzeichnet, dass** die Gasblasengeschwindigkeitsbestimmungsvorrichtung (26, 27, 28) eine Beleuchtungsvorrichtung (28) aufweist, wobei die Beleuchtungsvorrichtung (28) Licht annähernd parallel oder antiparallel zu einer Mittelachse (18) der Fluidleitung (13) in die Fluidleitung (13) einstrahlt.

5. Aufbereitungsvorrichtung nach Anspruch 4, **dadurch gekennzeichnet, dass** die Gasblasengeschwindigkeitsbestimmungsvorrichtung (26, 27, 28) eine Kamera (26) zur Aufnahme von zeitlich aufeinander folgenden Bildern wenigstens eines Abschnitts (20) der Fluidleitung (13) aufweist.

6. Aufbereitungsvorrichtung nach Anspruch 5, **dadurch gekennzeichnet, dass** die Fluidleitung (13) in dem Abschnitt (20) transparent ist.

7. Aufbereitungsvorrichtung nach einem der Ansprüche 4 bis 6, **dadurch gekennzeichnet, dass** die Beleuchtungsvorrichtung (28) Licht im infraroten Bereich emittiert.

8. Aufbereitungsvorrichtung nach einem der Ansprüche 5 bis 7, **dadurch gekennzeichnet, dass** der Abschnitt (20) wenigstens einen Bereich umfasst, der von einer Mittelachse (18) der Fluidleitung (13) $\geq R/2^{1/2}$ entfernt ist, wobei R ein Radius der Fluidleitung (13) oder ein Abstand der Mittelachse (18) zu einer Wand (17) der Fluidleitung (13) ist.

9. Aufbereitungsvorrichtung nach einem der Ansprüche 4 bis 8, **dadurch gekennzeichnet, dass** die Gasblasengeschwindigkeitsbestimmungsvorrichtung (26, 27, 28) ein Computersystem (27) umfasst, auf dem insbesondere eine Fouriertransformation von zeitlich aufeinanderfolgenden aufgenommenen Bildern durchführbar ist, wobei insbesondere über eine Phasenkorrelation ein Bewegungsvektor der Gasblasen (15) ermittelbar ist.

10. Verwendung von Gasblasen (15) zur Bestimmung des Volumenstroms eines Aufbereitungsfluids (14) in einem Kanal (12) eines aufzubereitenden medizinischen Instruments (11), wobei der Volumenstrom anhand der Geschwindigkeit (u) der Gasblasen (15) bestimmt wird, **dadurch gekennzeichnet, dass** die Geschwindigkeit (u) der Gasblasen (15) über eine Bildverarbeitung von wenigstens zwei zeitlich aufeinander folgenden aufgenommenen Bildern eines Ausschnitts (20) der Fluidleitung (13) bestimmt wird, wobei zur Kontrasterhöhung Licht (21) auf die Gasblasen (15) geleuchtet wird, wobei die Leuchtrichtung annähernd parallel oder antiparallel zur Fließrichtung (16) der Flüssigkeit (14) ist.

11. Volumenstrombestimmungsmodul zur Verwendung in oder mit einer Aufbereitungsvorrichtung zum Reinigen und/oder Desinfizieren eines medizinischen Instruments (11) umfassend eine Fluidleitung (13) oder einen Abschnitt einer Fluidleitung (13), eine Blaseneinleitvorrichtung (28) zum Einleiten von Gasblasen (15) in die Fluidleitung (13) oder den Abschnitt der Fluidleitung (13), und einer Gasblasengeschwindigkeitsbestimmungsvorrichtung (26, 27, 28), **dadurch gekennzeichnet, dass** die Gasblasengeschwindigkeitsbestimmungsvorrichtung (26, 27, 28) eine Beleuchtungsvorrichtung (28) aufweist, wobei die Beleuchtungsvorrichtung (28) Licht annähernd parallel oder antiparallel zu einer Mittelachse (18) der Fluidleitung (13) in die Fluidleitung (13) einstrahlt.

**Claims**

1. A method for operating a reprocessing apparatus (10) for cleaning and/or disinfecting a medical instrument (11) which has at least one channel (12), wherein a fluid line (13) is provided, which is or will be connected to the at least one channel (12), wherein, in order to determine the volume flow of a liquid (14) flowing in the fluid line (13), gas bubbles (15) are or will be admitted into the liquid (14), wherein the speed (u) of the gas bubbles (15) is determined and the volume flow is extrapolated on the basis of the speed (u) of the gas bubbles (15), **characterized in that** the speed (u) of the gas bubbles (15) is determined by way of an image processing of at least two successively captured images of a section (20) of the fluid line (13), wherein, in order to increase the contrast, light (21) is shone onto the gas bubbles (15), wherein the light direction is approximately parallel or antiparallel to the flow direction (16) of the liquid (14).

2. The method according to Claim 1, **characterized in that** the speed (u) of the gas bubbles (15) is determined in the vicinity of a wall (17) of the fluid line (13), wherein, to this end, the speed (u) of the gas bubbles (15) is in particular determined at a distance of the middle (18) of the fluid line (13) from a wall (17), wherein the distance is $\geq R/2^{1/2}$, wherein R is the internal radius of the fluid line (13) or a distance of a central axis (18) from a wall (17) of the fluid line (13).

3. The method according to Claim 1 or 2, **characterized in that**, in order to determine the speed (u), a fast Fourier transform of the two successively captured images is performed, wherein a movement vector of the gas bubbles (15) is established in particular by way of a phase correlation.

4. A reprocessing apparatus for cleaning and/or disinfecting medical instruments (11), having a fluid container (22) for a reprocessing fluid (14) and a reprocessing device (10), wherein the reprocessing device comprises a fluid line (13), wherein the reprocessing device (10) has a reprocessing space (23), in which a medical instrument (11) can be or is introduced for reprocessing, wherein at least one channel (12) of the medical instrument (11) can be or is connected to the fluid line (13), wherein the fluid line (13) serves to transport the reprocessing fluid (14) to the at least one channel (12), wherein a bubble introducing apparatus (25) is provided, by means of which gas bubbles (15) can be introduced into the fluid line (13), wherein a gas bubble speed determining apparatus (26, 27, 28) is in addition provided, **characterized in that** the gas bubble speed determining apparatus (26, 27, 28) comprises an illumination apparatus (28), wherein the illumination apparatus (28) shines light into the fluid line (13) approximately parallel or antiparallel to a central axis (18) of the fluid line (13).

5. The reprocessing apparatus according to Claim 4, **characterized in that** the gas bubble speed determining apparatus (26, 27, 28) has a camera (26) for capturing successive images of at least a portion (20) of the fluid line (13).

6. The reprocessing apparatus according to Claim 5, **characterized in that** the fluid line (13) is transparent in the portion (20).

7. The reprocessing apparatus according to any one of Claims 4 to 6, **characterized in that** the illumination apparatus (28) emits light in the infrared range.

8. The reprocessing apparatus according to any one of Claims 5 to 7, **characterized in that** the portion (20) comprises at least one region which is $\geq R/2^{1/2}$ distant from a central axis (18) of the fluid line (13), wherein R is a radius of the fluid line (13) or a distance of the central axis (18) from a wall (17) of the fluid line (13).

9. The reprocessing apparatus according to any one of Claims 4 to 8, **characterized in that** the gas bubble speed determining apparatus (26, 27, 28) comprises a computer system (27), on which a fast Fourier transform of successively captured images can in particular be performed, wherein a movement vector of the gas bubbles (15) can in particular be established by way of a phase correlation.

10. Use of gas bubbles (15) for determining the volume flow of a reprocessing fluid (14) in a channel (12) of a medical instrument (11) which is to be prepared, **characterized in that** the volume flow is determined on the basis of the speed (u) of the gas bubbles (15), wherein the speed (u) of the gas bubbles (15) is determined by way of an image processing of at least two successively captured images of a section (20) of the fluid line (13), wherein, in order to increase the contrast, light (21) is shone onto the gas bubbles (15), wherein the light direction is approximately parallel or antiparallel to the flow direction (16) of the liquid (14).

11. A volume flow determining module for use in or with a reprocessing apparatus for cleaning and/or disinfecting a medical instrument (11), comprising a fluid line (13) or a portion of a fluid line (13), a bubble introducing apparatus (28) for introducing gas bubbles (15) into the fluid line (13) or the portion of the fluid line (13), and a gas bubble speed determining apparatus (26, 27, 28), **characterized in that** the gas bubble speed determining apparatus (26, 27, 28) comprises an illumination apparatus (28), wherein the illumination apparatus (28) shines light into the fluid line (13) approximately parallel or antiparallel to a central axis (18) of the fluid line (13).

## Revendications

1. Procédé de fonctionnement d'un dispositif de traitement (10) pour le nettoyage et/ou la désinfection d'un instrument médical (11), qui présente au moins un canal (12), une conduite de fluide (13) étant prévue, qui est ou sera reliée à l'au moins un canal (12), dans lequel, pour déterminer le débit volumique d'un liquide (14) s'écoulant dans la conduite de fluide (13), des bulles de gaz (15) sont introduites ou sont aptes à être introduites dans le liquide (14), la vitesse (u) des bulles de gaz (15) étant déterminée et le débit volumique étant déduit en utilisant la vitesse (u) des bulles de gaz (15), **caractérisé en ce que** la vitesse (u) des bulles de gaz (15) est déterminée par un traitement d'image d'au moins deux images prises successivement dans le temps d'une portion (20) de la conduite de fluide (13), de la lumière (21) étant projetée sur les bulles de gaz (15) pour augmenter le contraste, la direction d'éclairage étant approximativement parallèle ou antiparallèle à la direction d'écoulement (16) du liquide (14).

2. Procédé selon la revendication 1, **caractérisé en ce que** l'on détermine la vitesse (u) des bulles de gaz (15) à proximité d'une paroi (17) de la conduite de fluide (13), en déterminant notamment à cet effet la vitesse (u) des bulles de gaz (15) à une distance allant du centre (18) de la conduite de fluide (13) jusqu'à une paroi (17), la distance étant $\geq R/2^{1/2}$, R étant le rayon intérieur de la conduite de fluide (13) ou une distance allant d'un axe central (18) jusqu'à une paroi (17) de la conduite de fluide (13).

3. Procédé selon la revendication 1 ou la revendication 2, **caractérisé en ce que**, pour déterminer la vitesse (u), on effectue une transformation de Fourier des images enregistrées successivement dans le temps, un vecteur de mouvement des bulles de gaz (15) étant notamment déterminé par une corrélation de phase.

4. Dispositif de traitement pour le nettoyage et/ou la désinfection d'instruments médicaux (11) ayant un réservoir de fluide (22) pour un fluide de traitement (14) et un appareil de traitement (10), le dispositif de traitement présentant une conduite de fluide (13), l'appareil de traitement (10) présentant un espace de traitement (23) dans lequel un instrument médical (11) peut être introduit ou est apte à être introduit pour le traitement, au moins un canal (12) de l'instrument médical (11) étant apte à être relié ou étant relié à la conduite de fluide (13), la conduite de fluide (13) servant à transporter le fluide de traitement (14) vers au moins un canal (12), un dispositif d'introduction de bulles (25) étant prévu, au moyen duquel des bulles de gaz (15) peuvent être introduites dans la conduite de fluide (13), un dispositif (26, 27, 28) de détermination de la vitesse des bulles de gaz étant en outre prévu, **caractérisé en ce que** le dispositif (26, 27, 28) de détermination de la vitesse des bulles de gaz présente un dispositif d'éclairage (28), le dispositif d'éclairage (28) émettant de la lumière dans la conduite de fluide (13) approximativement parallèlement ou antiparallèlement à un axe central (18) de la conduite de fluide (13).

5. Dispositif de traitement selon la revendication 4, **caractérisé en ce que** le dispositif (26, 27, 28) de détermination de la vitesse des bulles de gaz comporte une caméra (26) pour prendre des images successives dans le temps d'au moins une portion (20) de la conduite de fluide (13).

6. Dispositif de traitement selon la revendication 5, **caractérisé en ce que** la conduite de fluide (13) est transparente dans la portion (20).

7. Dispositif de traitement selon l'une des revendications 4 à 6, **caractérisé en ce que** le dispositif d'éclairage (28) émet de la lumière dans le domaine infrarouge.

8. Dispositif de traitement selon l'une des revendications 5 à 7, **caractérisé en ce que** la portion (20) comprend au moins une zone distante d'un axe central (18) de la conduite de fluide (13) $\geq R/2^{1/2}$, R étant un rayon de la conduite de fluide (13) ou une distance allant de l'axe central (18) jusqu'à une paroi (17) de la conduite de fluide (13).

9. Dispositif de traitement selon l'une des revendications 4 à 8, **caractérisé en ce que** le dispositif (26, 27, 28) de détermination de la vitesse des bulles de gaz comprend un système informatique (27) sur lequel peut notamment être réalisée une transformation de Fourier d'images enregistrées successivement dans le temps, un vecteur de mouvement des bulles de gaz (15) pouvant notamment être déterminé par une corrélation de phase.

10. Utilisation de bulles de gaz (15) pour déterminer le débit volumique d'un fluide de traitement (14) dans un canal (12) d'un instrument médical (11) à traiter, le débit volumique étant déterminé en utilisant la vitesse (u) des bulles de gaz (15), **caractérisée, en ce que** la vitesse (u) des bulles de gaz (15) est déterminée par un traitement d'image d'au moins deux images prises successivement dans le temps d'une portion (20) de la conduite de fluide (13), de la lumière (21) étant projetée sur les bulles de gaz (15) pour augmenter le contraste, la direction d'éclairage étant à peu près parallèle ou antiparallèle à la direction d'écoulement (16) du liquide (14).

11. Module de détermination du débit volumétrique destiné à être utilisé dans ou avec un dispositif de traitement pour le nettoyage et/ou la désinfection d'un instrument médical (11) comprenant une conduite de fluide (13) ou une portion d'une conduite de fluide (13), un dispositif d'introduction de bulles (28) pour introduire des bulles de gaz (15) dans la conduite de fluide (13) ou la portion de la conduite de fluide (13), et un dispositif (26, 27, 28) de détermination de la vitesse des bulles de gaz, **caractérisé en ce que** le dispositif (26, 27, 28) de détermination de la vitesse des bulles de gaz comprend un dispositif d'éclairage (28), le dispositif d'éclairage (28) projetant de la lumière dans la conduite de fluide (13) approximativement parallèlement ou antiparallèlement à un axe central (18) de la conduite de fluide (13).

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110097248 A1
- US 20100078047 A1

- WO 2011149539 A1